# FASCICULE DE BREVET EUROPEEN

(11) **EP 1 658 494 B1**
(45) Date de publication et mention de la délivrance du brevet: **12.10.2011**
(21) Numéro de dépôt: 04786344.4
(22) Date de dépôt: 23.08.2004
(51) Int. Cl.: G01N 33/50, G01N 33/68, G01N 33/569

(54) **METHODE DE TITRAGE IN VITRO D'UN ATNC ET SON APPLICATION A UNE METHODE D'EVALUATION ET/OU DE CONTROLE D'UN PROCEDE D'OBTENTION D'UN PRODUIT BIOLOGIQUE**
VERFAHREN ZUR IN-VITRO-TITRATION EINES NCTA UND ANWENDUNG DAVON IN EINEM VERFAHREN ZUR BEWERTUNG UND/ODER ÜBERWACHUNG EINES HERSTELLUNGSVERFAHRENS FÜR EIN BIOLOGISCHES PRODUKT
METHOD FOR THE IN VITRO TITRATION OF AN NCTA AND APPLICATION THEREOF IN A METHOD FOR THE EVALUATION AND/OR MONITORING OF A BIOLOGICAL PRODUCT PRODUCTION METHOD

(30) Priorité: 25.08.2003 FR 0310126
(43) Date de publication de la demande: 24.05.2006
(73) Titulaire: Laboratoire Français du Fractionnement et des Biotechnologies, 91940 Les Ulis (FR)
(72) Inventeur: AUBIN, Jean-Thierry, F-75003 Paris (FR); FLAN, Benoît, F-91470 Limours (FR)
(74) Mandataire: Hirsch & Associés
(86) Numéro de dépôt international: PCT/FR2004/002179
(87) Numéro de publication internationale: WO 2005/022148

(56) Documents cités:
- US-B1- 6 605 445
- VILETTE D ET AL: "Ex vivo propagation of infectious sheep scrapie agent in heterologous epithelial cells expressing ovine prion protein" PROCEEDINGS OF THE NATIONAL ACADEMY OF SCIENCES OF THE UNITED STATES, vol. 98, no. 7, 27 mars 2001 (2001-03-27), pages 4055-4059, XP002280772 March 27, 2001 ISSN: 0027-8424
- SABUNCU ELIFSU ET AL: "PrP polymorphisms tightly control sheep prion replication in cultured cells." JOURNAL OF VIROLOGY, vol. 77, no. 4, février 2003 (2003-02), pages 2696-2700, XP002315297 ISSN: 0022-538X
- LAUDE H ET AL: "New in vivo and ex vivo models for the experimental study of sheep scrapie: development and perspectives" COMPTES RENDUS - BIOLOGIES, ELSEVIER, PARIS, FR, vol. 325, no. 1, janvier 2002 (2002-01), pages 49-57, XP004334087 ISSN: 1631-0691
- ARCHER FABIENNE ET AL: "Cultured peripheral neuroglial cells are highly permissive to sheep prion infection." JOURNAL OF VIROLOGY, vol. 78, no. 1, janvier 2004 (2004-01), pages 482-490, XP002315320 ISSN: 0022-538X
- STENLAND CHRISTOPHER J ET AL: "Partitioning of human and sheep forms of the pathogenic prion protein during the purification of therapeutic proteins from human plasma." TRANSFUSION (BETHESDA), vol. 42, no. 11, novembre 2002 (2002-11), pages 1497-1500, XP002280769 ISSN: 0041-1132 (ISSN print)
- VEY M ET AL: "Purity of spiking agent affects partitioning of prions in plasma protein purification" BIOLOGICALS, vol. 30, no. 3, septembre 2002 (2002-09), pages 187-196, XP002280770 ISSN: 1045-1056
- LAU W ET AL: "Polymerase chain reaction based assessment of leukoreduction efficacy using a cytomegalovirus DNA transfected human T-cell line." JOURNAL OF CLINICAL VIROLOGY: THE OFFICIAL PUBLICATION OF THE PAN AMERICAN SOCIETY FOR CLINICAL VIROLOGY. NETHERLANDS 20 AUG 1998, vol. 11, no. 2, 20 août 1998 (1998-08-20), pages 109-116, XP002280771 ISSN: 1386-6532
- MACGREGOR I: "Prion protein and developments in its detection" TRANSFUSION MEDICINE, OXFORD, GB, vol. 11, no. 1, février 2001 (2001-02), pages 3-14, XP002220702

## Description

La présente invention concerne une méthode de titrage *in vitro* d'un agent transmissible non conventionnel (ATNC) utilisant des lignées cellulaires transgéniques, son application à une méthode d'évaluation et/ou de contrôle in vitro de l'efficacité d'un procédé d'obtention ou de traitement d'un produit biologique ou d'une ou plusieurs étapes d'un tel procédé pour l'élimination d'un ATNC et son application à une méthode d'évaluation et/ou de contrôle in vitro d'une procédure de décontamination.

Les encéphalopathies spongiformes transmissibles (EST) regroupent un ensemble de maladies génétiques ou acquises caractérisées par une dégénérescence du système nerveux central (SNC), connues chez l'être humain comme étant, entre autres, la maladie de Creutzfeld-Jacob (MCJ) et affectant également de nombreux mammifères, particulièrement les ovins et les bovins (tremblante du mouton et encéphalopathie spongiforme bovine). Les propriétés de l'agent étiologique de ces maladies le classent dans ce que l'on appelle les "agents transmissibles non conventionnels" (ATNC). L'agent causal n'est pas connu. Mais la signature de la maladie est la présence d'une protéine extracellulaire, appelée protéine prion (PrP), qui est transformée au cours de la maladie en une forme insoluble, résistante aux protéases telles que la protéinase K, et qui s'accumule dans les cellules, provoquant la mort de celles-ci. Cette forme anormale pathogène de la PrP, appelée PrPsc, résulte d'une modification de la conformation de la protéine prion PrP. Il n'a pas été mis en évidence de modification de l'expression du gène codant pour la PrP, ni d'altération de sa traduction (P. Brown, Transfusion, 41, 4333-436, 2001 et D. Vôlkel et al, Transfusion, 41, 441-448, 2001).

La transgénèse in vitro ou in vivo contribue largement à la connaissance des EST. Ainsi, les souris dont le gène codant pour la PrPsc a été inactivé sont très fortement résistantes à l'infection expérimentale. Inversement, des souris et des cultures cellulaires exprimant (ou sur-exprimant) des transgènes pathologiques, clonés à partir du gène de la PrP de sujets souffrant d'EST familiale, ou des xeno-gènes, miment les maladies héréditaires correspondantes ou sont sensibles à un innoculum provenant de sujets infectés appartenant à la même espèce que les transgènes. Les lignées cellulaires transgéniques sont également utilisées comme modèle in vitro pour la recherche de molécules interagissant avec la trans-conformation de la PrP en PrPsc.

Les données actuellement disponibles ne permettent pas de démontrer que l'agent transmissible responsable des EST se trouve sous une forme infectante dans le sang et les dérivés sanguins (voir référence précédente à P. Brown). Toutefois, on ne peut conclure à son absence, cette incertitude résultant, d'une part, de la probable concentration très faible dans le sang et, d'autre part, de la très longue période d'incubation de la maladie, lorsqu'elle est installée.

En outre, la surprenante résistance de l'ATNC empêche le recours à des procédés d'inactivation classiquement mis en oeuvre, tels que le traitement solvant/détergent Tween-TNBP, qui ont fait la preuve de leur efficacité pour réduire la charge virale des dérivés sanguins, tels que les protéines cryoprécipitables du plasma (facteur VIII, facteur von Willebrand etc.). Ceux-ci se verraient en effet complètement détruits par les procédés connus pour réduire l'infectiosité, par exemple en présence de NaOH 1M.

Etant donné que l'obtention ou le traitement de produits biologiques, tels que les protéines plasmatiques de la coagulation, doit incorporer des étapes d'élimination/inactivation virales en vue d'un usage thérapeutique, l'industrie pharmaceutique des médicaments dérivés du sang cherche aujourd'hui à évaluer le risque théorique de transmission du variant de la MCJ par les produits dérivés du sang.

Il est donc nécessaire de mettre au point une méthode de titrage sensible, spécifique et rapide, pouvant être mise en oeuvre pour évaluer le degré d'élimination de l'ATNC que peut apporter une étape ou une succession d'étapes mises en oeuvre dans le cadre d'un procédé de purification de produits biologiques ou dans le cadre d'une procédure de décontamination d'un matériel, notamment d'un matériel réutilisable.

Actuellement, la méthode classiquement mise en oeuvre utilise une méthode de titrage in vivo avec une lignée sensible de hamsters dorés, par injection intracérébrale de différentes dilutions d'un produit à tester chargé en prion pathogène. Selon le nombre d'animaux atteints dans les différents groupes correspondant aux dilutions effectuées, on peut calculer un titre infectieux et établir le quotient de réduction d'un procédé donné à partir d'une référence non traitée. Cependant, cette méthode présente l'inconvénient d'être longue, coûteuse et peu compatible avec un développement à l'échelle industrielle dont on veut connaître rapidement l'efficacité vis-à-vis de l'élimination du prion.

Sabuncu et al. (Journal of Virology, Feb. 2003, p. 2696-2700) décrit la détermination du titre infectieux d'extraits cellulaires infectées par titration limite *in vivo* dans des souris transgéniques TgOv exprimant l'allèle VRQ de la PrP ovine.

En outre, il est souvent nécessaire d'introduire une étape de concentration de l'agent infectieux de façon à augmenter la sensibilité des méthodes de titrage. Or, toutes les procédures de concentration d'agents infectieux responsables de l'EST co-purifient de la PrPsc.

Par ailleurs, des méthodes connues sont disponibles pour le titrage *in vitro* des agents infectieux des EST. Elles consistent en la détection de la PrPsc par la technique de Western-Blot (Ironside J.W. et al, J. of Thrombosis and Haemostasis, 1, 1479-1486, 2003) ou d'ELISA. Ces techniques nécessitent une digestion préalable de l'échantillon à analyser par la Protéinase K, ou une dénaturation par des agents chaotropiques pour distinguer la protéine pathologique (PrPsc) de la protéine normale (PrP). Une autre méthode de titrage a récemment été développée basée sur l'utilisation des anticorps spécifiques de la PrPsc ne reconnaissant pas la PrP.

Les titrages d'infectiosité liés aux ATNC s'effectuent par inoculation intracérébrale d'animaux de laboratoire, tels que des rongeurs qui ont préalablement permis l'adaptation de souches d'EST infectant d'autres espèces animales, ou de souris transgéniques exprimant une PrP de la même espèce que la source d'infectiosité.

Certains auteurs ont comparé la limite de détection des techniques immuno-chimiques de détection de la PrPsc à celle des techniques *in vivo* et *in vitro* (inoculation de cultures cellulaires transgéniques), dans un but de diagnostic. A titre d'exemple, on peut citer le brevet US 6 150 583 où sont prévus des animaux transgéniques exprimant la PrP épitope marquée.

Dans le cas des virus classiques, les techniques de détection des agents infectieux peuvent se faire de manière directe, tel que le titrage de l'infectiosité *in vitro* et *in vivo*, et de manière indirecte par les étapes d'amplification en chaîne par polymérase de génomes viraux, inoculation *in vivo* suivie de la mise en évidence de séroconversions vis-à-vis d'agents viraux caractérisés, et détection immuno-chimique ou moléculaire de marqueurs associés à une pathologie.

L'évaluation de techniques directes par rapport aux techniques indirectes est un pré-requis à leur utilisation expérimentale à des fins réglementaires. Ainsi, on distingue les virus pertinents dont le titrage d'infectiosité *in vitro* est validé (une souche virale adaptée sur une lignée cellulaire continue sensible et permissive), des virus modèles, plus proches parents d'un agent pathogène dont la propagation *in vitro* n'est pas reproductible.

A ce jour, la validation expérimentale, à l'égard des ATNC, des étapes d'inactivation virale incluses dans des procédés d'obtention ou de purification de produits biologiques ou des étapes de décontamination de matériel, ne peut être effectuée que de façon directe *in vivo* par inoculation intracérébrale par exemple de rongeurs de laboratoire ou de souris transgéniques, ou de façon indirecte *in vitro* par détection immuno-chimique de la PrPsc. En effet, aucun acide nucléique n'est présent dans le matériel infectieux et la PCR ne peut donc être utilisée. De plus, la protéine anormale in vivo ne provoque pas la formation d'anticorps spécifiques. Il faut donc recourir à des anticorps monoclonaux.

Pour pallier les inconvénients des méthodes précédentes de détection et de titrage des ATNC dans des produits biologiques, la Demanderesse a montré de manière surprenante, malgré la méconnaissance encore importante du comportement des ATNC, que l'utilisation de lignées cellulaires transgéniques supportant la réplication d'un ATNC permet la mise au point d'une méthode de titrage de cet ATNC. Cette méthode de titrage peut être notamment utilisée à des fins de validation de l'efficacité, vis-à-vis de l'élimination de l'ATNC considéré, de procédés d'obtention ou de traitement de produits biologiques, tels que, par exemple, les protéines issues du fractionnement du plasma sanguin, les aliments, les produits cosmétiques ou, plus généralement, tout produit présentant un risque pour l'environnement (par exemple des farines animales). Elle peut également être utilisée pour évaluer l'efficacité de procédures de décontamination de matériel, comme par exemple de colonnes de chromatographie.

L'application de la méthode de titrage d'un ATNC selon l'invention permet la détermination du titre absolu en ATNC présent dans une matrice biologique.

Dans le cadre de l'invention, le terme "ATNC" représente tout ATNC, tels que ceux responsables chez l'être humain de la MCJ familiale ou sporadique, de la maladie du Kuru ou du variant MCJ mis en évidence chez des sujets jeunes, ou bien encore ceux responsables chez les animaux de la tremblante ovine ou de l'encéphalopathie spongiforme bovine ou féline.

L'invention concerne donc une méthode de titrage *in vitro* d'un ATNC dans un produit biologique susceptible d'être infecté par cet ATNC, caractérisée en ce que des cellules transgéniques stables supportant la réplication dudit ATNC sont mises en contact avec l'échantillon biologique puis cultivées pendant un ou plusieurs passages pour amplifier la quantité d'ATNC présente dans ledit produit biologique par réplication dudit ATNC.

Le choix spécifique d'une lignée cellulaire transgénique permettant de mettre en oeuvre l'invention, répondant également aux exigences et critères définis ci-après, permet de répondre à la nécessité de quantifier l'infectiosité liée aux ATNC sur une gamme d'environ 4 log, c'est-à-dire de 1 à 10000 unités infectieuses in vitro. Ceci peut, par exemple, permettre de satisfaire aux critères de validation de l'efficacité des procédés d'obtention de produits biologiques vis-à-vis de l'élimination des ATNC.

L'utilisation de telles lignées cellulaires transgéniques dans la méthode de titrage selon l'invention présente de nombreux avantages, notamment celui de diminuer considérablement la durée de la quantification d'un ATNC présent dans le produit biologique, les résultats de la méthode de titrage selon l'invention étant correctement corrélés avec les méthodes *in vivo* de référence. On obtient typiquement des résultats de titrage en environ 1 mois et demi, alors que ceux de l'art antérieur utilisant les hamsters dorés par inoculation intracérébrale *in vivo*, sont obtenus en 1 an dans les cas les plus favorables et au prix d'examens complémentaires histopathologiques fastidieux pour définir le statut (infecté/non infecté) des animaux. Par ailleurs, ces lignées cellulaires offrent une réponse de mesure de titrage amplifiable, reproductible et la manifestation du pouvoir infectieux de l'ATNC qui n'est pas révélée par immuno-chimie. Elle présente aussi l'avantage d'éviter l'expérimentation sur de nombreux animaux et leur euthanasie.

De telles cellules transgéniques stables, permettant de mettre en oeuvre l'invention, répondent aux exigences et critères suivants.

Etant donné que les cellules nerveuses (neurones, cellules dendritiques) ne sont pas nécessairement les meilleurs substrats de transgénèse à des fins de titrage *in vitro*, du fait de leur mauvaise adaptation en culture *in vitro*, on établit des lignées cellulaires transgéniques par combinaison d'un type de cellules spécifiques et d'un transgène particulier, par des techniques connues, nécessaire pour fournir un environnement idéal à la réplication de l'ATNC.

Vilette et al. (PNAS, Mars 2001, 98(7), 4055-4059) ont montré que des cellules épithéliales de lapin, transgéniques et stables, exprimant la PrP ovine (transgène), sont capables de répliquer la PrPsc ovine après infection par des extraits contenant de la PrPsc ovine. Le modèle cellulaire qu'ils ont construit, la lignée Rov9, exprime de façon inductible la PrP exogène, ce qui garantit son maintien lors de la période d'incubation nécessaire à l'accumulation de la PrPsc dans ces cellules mises en contact avec un matériel infectieux. De telles cellules transgéniques stables supportant la réplication d'un ATNC sont susceptibles d'être utilisées dans la méthode de titrage selon l'invention.

Archer et al. (Journal of Virology, Jan. 2004, p. 482-490) ont construit un autre type de lignée cellulaire stable supportant la réplication de la PrPsc. Leur modèle consiste en des cellules gliales murines exprimant la PrP ovine et supportant la réplication de la PrPsc ovine, les cellules MovS. Ces cellules se sont révélées être particulièrement satisfaisantes dans la méthode de titrage selon l'invention. On recherche également d'autres critères pour qu'une lignée cellulaire transgénique soit avantageusement utilisée comme substrat pour le titrage *in vitro* d'ATNC. Ces critères sont les suivants :
- l'adéquation entre la vitesse de réplication ou multiplication des cellules et la durée d'incubation nécessaire à la mise en évidence d'une augmentation de la PrPsc dans les cellules infectées ;
- la stabilité des cellules après leur mise en contact avec l'agent infectieux qui peut être mise en évidence par l'absence de cytotoxicité de l'accumulation de la PrPsc dans les cellules ;
- leur bonne sensibilité à l'infection, par exemple une multiplicité d'infection faible pour obtenir une accumulation de la PrPsc dans les cellules exposées à l'agent infectieux.

Conformément à l'invention les cellules transgéniques stables supportant la réplication de l'ATNC sont mises en contact, par des méthodes connues en soi, avec le produit biologique susceptible d'être infecté par cet ATNC ou avec un matériel infectieux contenant l'ATNC en tant que matériel de référence. Dans le cadre de l'invention, le matériel infectieux représente un matériel biologique infectieux provenant notamment d'extraits de cerveaux d'animaux infectés par un ATNC, tels que de bovins ou d'ovins.

Ces cellules sont ensuite cultivées pendant un ou plusieurs passages pour permettre à l'ATNC de se répliquer. Un "passage" est le repiquage d'une partie des cellules d'une boîte de culture dans une autre boîte contenant du milieu de culture neuf. Chaque passage permet donc de diluer les cellules qui n'ont plus la place pour se diviser dans une autre boîte et le temps de culture dans une boîte permet à l'ATNC de se répliquer.

De manière avantageuse et conformément à l'invention, les cellules transgéniques peuvent être mises en contact avec au moins une dilution du produit biologique susceptible d'être infecté par l'ATNC dans une solution aqueuse biologiquement acceptable, en particulier avec plusieurs dilutions, tout particulièrement avec des dilutions sériées ou successives. Ces dilutions permettent d'affiner la quantification de l'infectiosité dans l'échantillon testé. Plusieurs réplicats de cellules transgéniques peuvent être mis en contact avec la même dilution du produit biologique.

Avantageusement, l'ATNC est détecté à chaque passage, en particulier par une méthode immunochimique connue de l'homme du métier, tout particulièrement par Western-Blot ou ELISA. L'étape de culture des cellules transgénique stables potentiellement infectées par le produit biologique, selon toute technique connue de l'homme du métier, est requise pour la réplication de l'ATNC donc pour amplifier une quantité d'ATNC insuffisante pour être détectée par des méthodes conventionnelles (ELISA, Western-Blot).

En particulier l'ATNC titré par la méthode selon l'invention est la protéine prion pathogène PrPsc, ovine, bovine ou humaine.

L'ensemble des résultats de Western-blot pour les différentes dilutions et les différents réplicats peut être analysé par une méthode statistique connue de l'homme du métier permettant d'établir un titre infectieux, comme par exemple la méthode de Spearman Kärber (Schmidt N.J., Emmous R.W., Diagnostic Procédures for viral, rickettsial and chlaveydial Infection, 1989, 6ème Edition).

De manière avantageuse, les cellules transgéniques stables sont des cellules épithéliales de lapin, en particulier des cellules épithéliales de lapin de la lignée Rov9 (Vilette *et al*.) ou des cellules gliales murines, en particulier des cellules gliales murines de la lignée MovS6 (Archer *et al.).*

Avantageusement, le produit biologique susceptible d'être contaminé par un ATNC est choisi dans le groupe constitué par les produits sanguins et leurs dérivés, les aliments, les produits cosmétiques et tout produit présentant un risque pour l'environnement.

L'invention concerne également l'application de la méthode de titrage selon l'invention à une méthode d'évaluation et/ou de contrôle *in vitro* d'un procédé d'obtention ou de traitement d'un produit biologique susceptible d'être contaminé par un agent transmissible non conventionnel (ATNC). Cette méthode d'évaluation et/ou de contrôle est caractérisée en ce qu'on applique au produit biologique, en amont et en aval dudit procédé, une méthode de titrage selon l'invention, telle que décrite précédemment, et en ce que l'on compare les deux valeurs de titre obtenues. Par comparaison entre les deux mesures, on détermine le degré d'élimination ou le facteur de réduction de l'ATNC.

En particulier, la méthode de titrage selon l'invention a la capacité d'être aisément applicable à tout type de procédé d'obtention ou de purification de produits biologiques, en particulier de produits sanguins, tels que les dérivés de plasma sanguins, utilisant par exemple les chromatographies ou la nanofiltration, en particulier les chromatographies décrites dans le brevet EP 0 359 593 et dans la demande de brevet WO 02092632.

Ainsi, la mise en oeuvre de la méthode de titrage selon l'invention permet d'évaluer et/ou de contrôler l'efficacité d'un procédé (ou d'une partie d'un procédé) d'obtention ou de traitement, voire de purification, de tout produit biologique, susceptible d'être contaminé par un ATNC, dans l'élimination de cet ATNC, grâce à un titrage utilisant des lignées cellulaires transgéniques spécifiques, favorisant la réplication de l'ATNC, mises en contact avec un matériel infectieux ou potentiellement infectieux contenant l'ATNC à tester. On mesure les quantités d'ATNC en amont et en aval du procédé (ou de la partie du procédé) dont on veut apprécier l'efficacité à l'égard de l'ATNC. Par comparaison des deux mesures, on détermine le degré d'élimination de l'agent pathogène. Ainsi, la mise en oeuvre de la présente méthode peut être effectuée au cours d'un procédé d'obtention d'un produit biologique ou dans le cadre d'un traitement d'élimination de l'ATNC suivant l'obtention du produit biologique.

L'invention concerne également l'application de la méthode de titrage selon l'invention à une méthode d'évaluation et/ou de contrôle in vitro d'une procédure de décontamination d'un matériel. Dans ce cas, on détermine le titre en ATNC d'un produit biologique contenant un ATNC au moyen de la méthode de titrage selon l'invention. On met ensuite en contact ce produit biologique infecté avec le matériel à décontaminer puis on applique la procédure de décontamination à ce matériel. Enfin, on détermine à nouveau le titre du produit biologique ayant subi la procédure de décontamination. Les deux mesures de titre effectuées en amont et en aval de la procédure de décontamination sont comparées pour évaluer l'efficacité de la procédure de décontamination.

Le produit biologique contenant un ATNC provient, par exemple, d'extraits de cerveaux d'animaux infectés par un ATNC, tels que de bovins ou d'ovins.

Le matériel peut, par exemple, être un matériel de purification, en particulier une colonne de chromatographie.

La procédure de décontamination peut, par exemple, être la sanitisation d'une colonne de chromatographie à l'aide de soude.

### Exemple 1 : Titrage d'infectiosité liée aux ATNC par culture cellulaire

Pour étudier la faisabilité d'un système de titrage in vitro de l'infectiosité liée aux ATNC, les cellules MovS6 (Archer F. *et al*. 2004) ont été sélectionnées comme cellules supportant la réplication des ATNC, et la souche de Scrapie PG127 adaptée aux souris transgéniques Tg301 a été utilisée comme source d'infectiosité (Vilotte JL et al., Journal of Virology, Vol. 75, n°13, p.5977-5984, 2001). L'inoculum consistait en un homogénat de cerveaux de souris infectées à 100 mg/ml. Les cellules étaient cultivées en milieu DMEM + HamF-12 complémenté en glutamine et sérum de veau foetal. Les plaques de titrages étaient préparées 24 heures avant l'inoculation. Les cellules étaient ensemencées à raison de 40.000 cellules par puits pour un format 96 puits (expérience n°1), et 100.000 cellules pour des plaques de 24 puits (expérience n° 2 et 3). Des dilutions sériées de l'homogénat de cerveaux de souris étaient préparées avec du milieu de culture, les pas de dilutions étaient de 10 en 10 (expériences n°1 et n°2), ou de 5 en 5 (expérience n°3) . Pour chaque dilution de l'inoculum 5 puits de plaque de culture étaient infectés. Les cellules étaient mises en contact avec un certain volume d'inoculum (50 à 150 µl) pour une période allant de 12 heures à 4 jours. Le Tableau 1 récapitule les conditions expérimentales d'inoculation et d'expansion des cellules spécifiques à chaque expérience. L'inoculum était jeté et remplacé par du milieu de culture neuf puis les cellules étaient maintenues en culture jusqu'au premier passage au cours duquel un changement de format de plaque de culture était réalisé afin de garder la moitié (expérience n°1), ou la totalité des cellules infectées (expériences n° 2 et 3). Lors de la poursuite de la culture cellulaire, le milieu de culture était changé une fois par semaine et les cellules repiquées avec un rapport de 1 sur 10, permettant d'effectuer des analyses sur 90 % des cellules à chaque passage. Les cellules récupérées à chaque passage étaient congelées sous forme de culots cellulaires, et conservées à -80°C jusqu'à leur analyse par la méthode de Western Blot pour détecter la PrPsc. Chaque culot cellulaire était traité 2 heures à 37°C par la Benzonase (250 unités) dans un volume de 50 µl. Les lysats cellulaires étaient ensuite traités par la Protéinase K, dénaturés puis analysés par électrophorèse en gel de polyacrylamide en conditions dénaturantes (SDS-PAGE). Les protéines ayant migré dans le gel étaient transférées sur une membrane de nitrocellulose par électro-transfert. La PrPsc présente sur les membranes était détectée par incubation avec l'anticorps 6H4 (Prionics) puis un anticorps secondaire marqué (anticorps de chèvre dirigés contre des anticorps de souris). Les membranes marquées étaient révélées par chemi-luminescence. Un échantillon était considéré comme positif si le profil électrophorétique des trois formes de PrPsc glycosylée était visible sur les autoradiogramme. Pour chaque analyse par Western Blot, des témoins négatifs (cellules MovS6 non infectées) étaient traités en parallèle des échantillons. A chacun des passages cellulaires, l'ensemble des puits de plaques de culture était testé. Lorsque l'ensemble des réplicats de cultures cellulaires inoculées avec une dilution donnée de l'inoculum était retrouvé positif pour la PrPsc lors de deux passages successifs, ces cultures n'étaient plus testées lors des passages suivants. Le titre d'un échantillon était calculé à la fin de la culture cellulaire par la méthode de Spearman Kärber (Schmidt NJ and Emmons RW, Diagnostic Procedures for Viral, Rickettsial and Chlamydial Infection 1989, 6th Edition).

**Tableau 1. Conditions expérimentales des expériences de titrage in vitro de l'infectiosité liée aux ATNC.**

| Paramètres | Expérience n°1 | Expérience n°2 | Expérience n°3 |
|---|---|---|---|
| Format de culture pour l'inoculation | Microplaque 96 puits | Plaque 24 puits | Plaque 24 puits |
| Densité cellulaire d'ensemencement (cellules/puits) | 40 000 | 100 000 | 100 000 |
| Volume de l'inoculum (µl) | 50 | 1 100 (100 µl dilué dans 1 ml de milieu frais) | 150 |
| Durée mise en contact primaire (heures) | 12 | 96 | 24 |
| Ajout de milieu frais (ml) | 0,2 | 1 (inoculum jeté) | 1 |
| Incubation secondaire (heures) | 48 en présence d'inoculum dilué, puis 48 avec du milieu frais. | 24 | 72 |
| Pourcentage de cellules repiquées au 1^{er} passage (changement de format 6 puits) | 50 % | 100 % | 100 % |

### Résultats

### Expérience n°1

Les dilutions de l'homogénat de cerveaux de souris infectées par la souche de Scrapie PG127 testés étaient de 10⁻¹ à 10⁻⁶. Au passage n°3, aucun puits de culture cellulaire ne présentait de PrPsc. Au passage n°4, 100 % des puits ayant été inoculés avec les dilutions 10⁻¹ à 10⁻³ de l'inoculum étaient positif pour la PrPsc, et 75 % des cultures infectées par la dilution 10⁻⁴ étaient positives. Au passage n°5, 25% des puits inoculés à la dilution 10⁻⁵ étaient positifs, aboutissant à l'établissement d'un titre de 6,05 log de dose infectieuse 50% (TCID₅₀) par ml selon la méthode de Spearman Karber. Le titre du stock n'augmentait pas lors des passages cellulaires suivants.

### Expérience n°2

Le Tableau 2 résume les résultats obtenus lors de l'expérience n° 2, et permet de constater à nouveau l'évolution du pourcentage de positivité de chacun des réplicats de culture infecté par chacune des dilution de l'inoculum (de 10⁻³ à 10⁻⁷). On peut constater que le délai d'apparition de la PrPsc dans les cultures est inversement proportionnel à la dose d'infectiosité avec laquelle les cultures ont été inoculées. Ainsi les cultures ayant reçu la dilution 10⁻³ de l'inoculum présentaient de la PrPsc dès le passage n°2, alors qu'il fallait attendre un passage supplémentaire pour que 100 % des cultures ayant reçu la dilution 10⁻⁴ soient positifs. Le titre de l'inoculum calculé pour cette expérience était de 5,5 TCID₅₀/ml.

**Tableau 2. Résultats de l'expérience de titrage n°2.**

| Passage n° | Nombre de cultures infectées selon la dilution inoculée (puits infectés/puits inoculés) | | | | | | Titre (log TCID₅₀/ml) |
|---|---|---|---|---|---|---|---|
| | 10⁻³ | 10⁻⁴ | 10⁻⁵ | 10⁻⁶ | 10⁻⁷ | Témoin nég. | |
| 1 | 0/5 | 0/5 | 0/5 | 0/5 | 0/5 | 0/5 | 0 |
| 2 | 5/5 | 1/5 | 0/5 | 0/5 | 0/5 | 0/5 | 4,7 |
| 3 | 5/5 | 5/5 | 0/5 | 0/5 | 0/5 | 0/5 | 5,5 |
| 4 | NT | 5/5 | 0/5 | 0/5 | 0/5 | 0/5 | 5,5 |
| 5 | NT | NT | 0/5 | 0/5 | 0/5 | 0/5 | 5,5 |
| 6 | NT | NT | 0/5 | 0/5 | 0/5 | 0/5 | 5,5 |

| | | | | | | | |
|---|---|---|---|---|---|---|---|
| NT : Non testé, car 100 % positif pour les deux passages précédents. | | | | | | | |

### Expérience n°3

Le Tableau 3 résume les résultats obtenus lors de l'expérience n° 3. Pour cette expérience, le pas de dilution de l'inoculum était réduit à 1/5, et les dilutions testées étaient de 10⁻⁴ à 10^{-6,8}. Le titre maximum étaient obtenu au passage n° 6, et le titre calculé était de 6,43 TCID₅₀ /ml.

**Tableau 3. Résultats de l'expérience n°3.**

| Passage n° | Nombre de cultures infectées selon la dilution inoculée (puits infectés / puits inoculés) | | | | | | Titre (log TCID₅₀/ml) |
|---|---|---|---|---|---|---|---|
| | 10⁻⁴ | 10^{-4,7} | 10^{-5,4} | 10^{-6,1} | 10^{-6,8} | Témoin nég. | |
| 1 | 0/5 | 0/5 | 0/5 | 0/5 | 0/5 | 0/5 | 0 |
| 2 | 1/5 | 0/5 | 0/5 | 0/5 | 0/5 | 0/5 | 3,4 |
| 3 | 5/5 | 3/5 | 0/5 | 0/5 | 0/5 | 0/5 | 5,6 |
| 4 | 5/5 | 5/5 | 3/5 | 0/5 | 0/5 | 0/5 | 6,3 |
| 5 | NT | 5/5 | 3/5 | 0/5 | 0/5 | 0/5 | 6,3 |
| 6 | NT | NT | 4/5 | 0/5 | 0/5 | 0/5 | 6,4 |
| 7 | NT | NT | 4/5 | 0/5 | 0/5 | 0/5 | 6,4 |

| | | | | | | | |
|---|---|---|---|---|---|---|---|
| NT : Non testé, car 100% positif pour les deux passages précédents. | | | | | | | |

Les résultats de ces trois expériences réalisées démontrent la faisabilité d'un titrage *in vitro* en TCID₅₀ de l'infectiosité liée aux ATNC. A l'instar des titrages *in vitro* des virus conventionnels par infection de cultures cellulaires, les cellules MovS6 permettent la mise en évidence de l'accumulation de la PrPsc dans les cellules infectées, et permettent la détection de quantité de PrPsc indétectable par des techniques immuno-chimiques dans l'inoculum avant son amplification par les cellules. Les conditions opératoires du système de titrage qui ont été mises au point ont été optimisées pour obtenir une sensibilité satisfaisante, supérieure à celle de la détection directe de la PrPsc dans l'inoculum par la technique de Western Blot, et qui garantit une marge de quantification supérieure ou égale à 4 log. Lorsque l'on compare les résultats obtenus lors des différentes expériences, on observe que les modalités de l'infection des cellules influent légèrement les titres de l'inoculum calculés, mais que d'une façon générale ces titres sont cohérents. En effet, compte tenu de la dilution de l'inoculum (100 µl) dans 1 ml de milieu de culture couvrant les cellules de l'expérience n°2, on peut considérer que ce titrage a été effectué avec des dilutions dix fois inférieures à celle de l'inoculum initial.

Ces expériences démontrent la supériorité du titrage *in vitro* de l'infectiosité liée aux ATNC par rapport au système de titrage classique *in vivo* nécessitant l'inoculation intracérébrale d'un nombre significatif de rongeurs de laboratoire, des délais d'obtention des résultats se comptant en mois, et généralement la confirmation du statut de l'infection des animaux inoculés par examen histopathologique ou par la technique de Western Blot de leurs cerveaux. De plus, le système satisfait aux prescriptions actuelles en matière de limitation des essais avec des animaux de laboratoire au profit de méthodes de substitution par culture cellulaire par exemple. Enfin, le système proposé peut être mis en oeuvre dans un laboratoire P2 contrairement aux essais avec animaux qui nécessite un confinement en laboratoire P3 plus onéreux et plus complexe.

### Exemple 2 : Calcul d'un facteur de réduction associé à une étape de purification d'un médicament biologique susceptible d'être contaminé par un ATNC

Pour cette expérience, une étape de nanofiltration était choisie comme exemple d'une étape de procédé de fabrication d'un produit biologique. Le filtre choisi était un filtre PLANOVA 15 N (ASAHI KASEI) d'une porosité de 15 nm et d'une surface de 0,01 m². Le produit à filtrer choisi était de l'albumine humaine à 0,2 g/l, dans du tampon citrate trisodique dihydrate 0,01 M, glycine 0,12 M, L-lysine (monochlorure) 0,016 M, chlorure de calcium dihydrate 0,001M, chlorure de sodium 0,17 M, osmolarité 490-510 mosmol/kg, pH 6,90-7,10. La nanofiltration était effectuée sous une pression de 500 ± 100 mbar, à 25 °C. Le filtre était équilibré avant la nanofiltration du produit avec 40 ml de tampon de dilution de l'albumine humaine. Un volume de 30 ml de produit à filtré, expérimentalement surchargé à 4 % avec de l'homogénat de cerveaux de souris infectées par la souche de Scrapie PG127 était filtré, puis un volume de 10 ml de tampon d'équilibrage était filtré pour faire sortir le produit du filtre. Deux expériences de nanofiltration étaient réalisées afin de conforter la reproductibilité de l'étape. Le produit à filtrer surchargé expérimentalement était préparé en quantité suffisante pour prélever une fraction aliquote destinée à quantifier l'infectiosité présente dans le produit avant la nanofiltration. Un volume total d'environ 40 ml de filtrat était récupéré en aval de la nanofiltration, et titré afin de déterminer la quantité d'infectiosité dans le produit après la nanofiltration. Le matériel de départ surchargé expérimentalement, ainsi que le produit filtré étaient dilués au 1/3 dans du milieu de culture avant d'être conservés à -80°C en l'attente de leur titrage. Les échantillons étaient titrés selon les conditions expérimentales de l'expérience n°3 décrite dans le premier exemple. Pour les échantillons de filtrats dont on attendait qu'ils ne présentent pas ou très peu d'infectiosité résiduelle, la plus petite dilution de l'échantillon non cytotoxique a été incoculée sur dix réplicats, et les dilutions suivantes sur 5 réplicats comme précédemment décrit.

Le Tableau 4 résume les résultats des titrages des quatre échantillons générés durant cette expérience de nanofiltration.

Les échantillons de matériel de départ surchargé présentaient des titres de 4,67 log TCID₅₀ /ml pour les essais 1 et 2. Aucune infectiosité résiduelle n'était détectée dans les échantillons de filtrats. La charge de ces échantillons était calculée selon la loi de Poisson à 0,28 log TCID₅₀ /ml à partir du volume de la plus petite dilution de l'échantillon inoculée. La charge totale présente dans les échantillons était calculée en multipliant leur charge par leur volume respectif, et le facteur de réduction était calculé en divisant la charge présente dans le matériel de départ, ou échantillon initial, avant la nanofiltration par celle présente dans les filtrats. Le facteurs de clairance étaient calculés à partir de la quantité calculée d'infectiosité ajoutée dans la surcharge expérimentale. La différence entre le facteur de clairance et le facteur de réduction permet de mettre en évidence, quand elle est supérieure à un log, l'interférence du produit de départ sur la capacité du système de titrage à détecter de l'infectiosité dans ce produit. Le Tableau 5 résume ces calculs. Les facteurs de réduction associés à l'élimination de l'infectiosité présente dans le broyat de cerveau ayant servi à surcharger le matériel de départ étaient de ≥ 4,24 et ≥ 4,22 log pour les essais 1 et 2 respectivement. Aucune interférence du matériel de départ n'était mise en évidence par le calcul des facteurs de clairance.

**Tableau 4. Résultats des titrages des échantillons générés lors de l'évaluation d'une étape de nanofiltration.**

| Echantillon | Dernières dilutions positives | nombre de cultures infectées/nombre de cultures inoculées | Titre (log TCID₅₀/ml) |
|---|---|---|---|
| Echantillon initial essai 1 | 1/3 125 1/15 625 | 4/5 1/5 | 4,67 |
| Filtrat essai 1 | Pur | 0/10 | < 0,28^{*} |
| Echantillon initial essai 2 | 1/3 125 1/15 625 | 3/5 2/5 | 4,67 |
| Filtrat essai 2 | Pur | 0/10 | < 0,28^{*} |

| | | | |
|---|---|---|---|
| * : pas d'infectiosité détectée : limite de détection calculée par la loi de Poisson. | | | |

**Tableau 5. Calcul des facteurs de réduction associés à l'étape de nanofiltration 15 nm évaluée par titrage in vitro.**

| Echantillon | Titre (log TCID₅₀/ml) | Volume (ml) | Charge totale (log TCID₅₀) | Facteur de Clairance (log)^{a} | Facteur de Réduction (log)^{b} |
|---|---|---|---|---|---|
| Stock de départ essai 1 | 6,43 | 1,21^{c} | 6,51 | | |
| Echantillon initial essai 1 | 4,67 | 30 | 6,14 | | |
| Filtrat essai 1 | < 0,28^{d} | 41,9 | < 1,90 | ≥ 4,61 | ≥ 4,24 |
| Stock de départ essai 2 | 6,43 | 1,28^{c} | 6,54 | | |
| Echantillon initial essai 2 | 4,67 | 30 | 6,14 | | |
| Filtrat essai 2 | < 0,28^{d} | 44,3 | < 1,92 | ≥ 4, 62 | ≥ 4,22 |

| | | | | | |
|---|---|---|---|---|---|
| a : calculé à partir de la charge totale dans la surcharge expérimentale. b : calculé à partir de la charge initiale mesurée dans le matériel de départ. c : volume prenant en compte le prélèvement de la fraction aliquote destinée au titrage de l'échantillon initial. d : pas d'infectiosité détectée : limite de détection calculée par la loi de Poisson. | | | | | |

## Revendications

1. Méthode de titrage *in vitro* d'un agent transmissible non conventionnel (ATNC) dans un produit biologique, dans laquelle des cellules transgéniques stables supportant la réplication dudit ATNC sont mises en contact avec ledit produit biologique puis cultivées pendant un ou plusieurs passages pour amplifier la quantité d'ATNC présente dans ledit produit biologique par réplication dudit ATNC.

2. Méthode de titrage selon la revendication 1, dans laquelle les cellules transgéniques stables sont cultivées en présence d'au moins une dilution du produit biologique dans une solution aqueuse biologiquement acceptable.

3. Méthode de titrage selon la revendication 1 ou 2, dans laquellle l'ATNC est détecté à chaque passage.

4. Méthode de titrage selon la revendication 3, dans laquelle l'ATNC est détecté à chaque passage par une méthode immunochimique.

5. Méthode de titrage selon la revendication 4 dans laquelle l'ATNC est détecté à chaque passage par Western-blot.

6. Méthode de titrage selon la revendication 5, dans laquelle le titre de l'ATNC est mesuré en appliquant une méthode statistique.

7. Méthode de titrage selon la revendication 6, dans laquelle le titre de l'ATNC est mesuré en applicant la méthode de Spearman-Kärber.

8. Méthode de titrage selon la revendication 4, dans laquelle l'ATNC est détecté à chaque passage par ELISA.

9. Méthode de titrage selon l'une quelconque des revendications 1 à 8, dans laquelle l'ATNC correspond à une forme pathogène de la prion protéine PrPsc.

10. Méthode de titrage selon l'une quelconque des revendications 1 à 9, dans laquelle les cellules transgéniques stables sont des cellules épithéliales de lapin.

11. Méthode de titrage selon les revendications 9 et 10, dans laquelle les cellules transgéniques stables sont des cellules épithéliales de lapin de la lignée Rov9.

12. Méthode de titrage selon l'une quelconque des revendications 1 à 9, dans laquelle les cellules transgéniques stables sont des cellules gliales murines.

13. Méthode de titrage selon les revendications 9 et 12, dans laquelle les cellules transgéniques stables sont des cellules gliales murines de la lignée MovS6.

14. Méthode de titrage selon l'une quelconque des revendications 1 à 13, dans laquelle le produit biologique est choisi parmi le groupe constitué par les produits sanguins et leurs dérivés, les aliments, les produits cosmétiques et les produits présentant un risque pour l'environnement.

15. Méthode d'évaluation et/ou de contrôle *in vitro* d'un procédé d'obtention ou de traitement d'un produit biologique susceptible d'être contaminé par un ATNC, dans laquelle on applique audit produit biologique, en amont et en aval dudit procédé, une méthode de titrage selon l'une quelconque des revendications 1 à 14, et on compare les deux valeurs de titre obtenues.

16. Application d'une méthode d'évaluation et/ou de contrôle selon la revendication 15 à un procédé de purification de produits sanguins et de leurs dérivés.

17. Application selon la revendication 16, dans laquelle le procédé de purification représente les chromatographies ou la nanofiltration.

18. Méthode d'évaluation et/ou de contrôle *in vitro* d'une procédure de décontamination d'un matériel, dans laquelle on met en contact ledit matériel à décontaminer avec un produit biologique contenant un ATNC et ce qu'on applique audit produit biologique, en amont et en avale de ladite procédure, une méthode de titrage selon l'une quelconque des revendications 1 à 14, et on compare les deux valeurs de titre obtenues.

## Claims

1. An in vitro titration method of a non conventional transmissible agent (NCTA) in a biological product, wherein stable transgenic cells tolerating the replication of said NCTA are contacted with the said biological product, then cultured in one or more passages in order to amplify the amount of NCTA present in the said biological product by replication of the said NCTA.

2. The titration method according to claim 1, wherein the stable transgenic cells are cultured in the presence of at least one dilution of the biological product in an aqueous biologically acceptable solution.

3. The titration method according to claim 1 or 2, wherein the NCTA is detected at each passage.

4. The titration method according to claim 3, wherein the NCTA is detected at each passage by an immunochemical method.

5. The titration method according to claim 4, wherein the NCTA is detected at each passage by Western blot.

6. The titration method according to claim 5, wherein the titre of NCTA is measured by applying a statistical method.

7. The titration method according to claim 6, wherein the titre of NCTA is measured by applying the Spearman-Kärber method.

8. The titration method according to claim 4, wherein the NCTA is detected at each passage by ELISA.

9. The titration method according to any one of claims 1 to 8, wherein the NCTA corresponds to a pathogenic form of the prion protein PrPsc.

10. The titration method according to any one of claims 1 to 9, wherein the stable transgenic cells are rabbit epithelial cells.

11. The titration method according to claims 9 and 10, wherein the stable transgenic cells are rabbit epithelial cells from the Rov9 cell line.

12. The titration method according to any one of claims 1 to 9, wherein the stable transgenic cells are mouse glial cells.

13. The titration method according to claims 9 and 12, wherein the stable transgenic cells are mouse glial cells from the MovS6 cell line.

14. The titration method according to any one of claims 1 to 13, wherein the biological product is selected from the group consisting of blood products and their derivatives, foodstuffs, cosmetics and products being a hazard for the environment.

15. An in vitro assessment and/or control method of a process for obtaining or treatment of a biological product susceptible to be contaminated with an NCTA, wherein the titration method according to any one of claims 1 to 14 is applied, upstream and downstream of the said process, to said biological product, and in that the both obtained titre values are compared.

16. Application of an assessment and/or control method according to claim 15 to a process of purification of blood products and their derivatives.

17. Application according to claim 16, wherein the purification process is chromatographies or the nanofiltration.

18. An in vitro assessment and/or control method of a material decontamination procedure, wherein the said material to be decontaminated is contacted with a biological product containing a NCTA and a method of titration according to any one of claims 1 to 14 is applied to the said biological product, upstream and downstream of the said procedure, and the both obtained titre values are compared.

## Patentansprüche

1. Verfahren zur In-vitro-Titration eines unkonventionellen übertragbaren Agens (NCTA) in einem biologischen Produkt, wobei stabile transgene Zellen, die die Replikation des NCTA tolerieren, mit dem biologischen Produkt in Kontakt gebracht werden, dann während einer oder mehrerer Passagen kultiviert werden, um die Menge an NCTA, die in dem biologischen Produkt vorhanden ist, durch Replikation des NCTA zu amplifizieren.

2. Verfahren zur Titration nach Anspruch 1, wobei die stabilen transgenen Zellen in Gegenwart mindestens einer Verdünnung des biologischen Produkts in einer biologisch akzeptablen wässrigen Lösung kultiviert werden.

3. Verfahren zur Titration nach Anspruch 1 oder 2, wobei das NCTA bei jeder Passage nachgewiesen wird.

4. Verfahren zur Titration nach Anspruch 3, wobei das NCTA bei jeder Passage durch ein immunochemisches Verfahren nachgewiesen wird.

5. Verfahren zur Titration nach Anspruch 4, wobei das NCTA bei jeder Passage durch Western-Blot nachgewiesen wird.

6. Verfahren zur Titration nach Anspruch 5, wobei der Titer des NCTA gemessen wird, indem ein statistisches Verfahren angewendet wird.

7. Verfahren zur Titration nach Anspruch 6, wobei der Titer des NCTA gemessen wird, indem das Spearman-Kärber-Verfahren angewendet wird.

8. Verfahren zur Titration nach Anspruch 4, wobei das NCTA bei jeder Passage durch ELISA nachgewiesen wird.

9. Verfahren zur Titration nach irgendeinem der Ansprüche 1 bis 8, wobei das NCTA einer pathogenen Form des Prionproteins PrPsc entspricht.

10. Verfahren zur Titration nach irgendeinem der Ansprüche 1 bis 9, wobei die stabilen transgenen Zellen Epithelzellen des Kaninchens sind.

11. Verfahren zur Titration nach den Ansprüchen 9 und 10, wobei die stabilen transgenen Zellen Epithelzellen des Kaninchens der Linie Rov9 sind.

12. Verfahren zur Titration nach irgendeinem der Ansprüche 1 und 9, wobei die stabilen transgenen Zellen murine Gliazellen sind.

13. Verfahren zur Titration nach den Ansprüchen 9 und 12, wobei die stabilen transgenen Zellen murine Gliazellen der Linie MovS6 sind.

14. Verfahren zur Titration nach einem der Ansprüche 1 und 13, wobei das biologische Produkt ausgewählt ist aus der Gruppe bestehend aus den Blutprodukten und deren Derivaten, den Nahrungsmitteln, den kosmetischen Produkten und den Produkten, die ein Risiko für die Umgebung darstellen.

15. Verfahren zur In-vitro-Bewertung und/oder -Überwachung eines Prozesses zum Erhalten oder zum Behandeln eines biologischen Produkts, das geeignet ist, mit einem NCTA kontaminiert zu werden, wobei auf das biologische Produkt stromaufwärts und stromabwärts des Prozesses ein Verfahren zur Titration nach irgendeinem der Ansprüche 1 bis 14 angewendet wird, und die beiden Werte der erhaltenen Titer verglichen werden.

16. Anwendung eines Verfahrens zur Bewertung und/oder zur Überwachung nach Anspruch 15 auf einen Prozess zur Reinigung von Blutprodukten und deren Derivaten.

17. Anwendung nach Anspruch 16, wobei der Prozess zur Reinigung aus Chromatographien oder Nanofiltration besteht.

18. Verfahren zur In-vitro-Bewertung und/oder -Überwachung eines Prozesses zur Dekontamination eines Materials, wobei das zu dekontaminierende Material mit einem biologischen Produkt in Kontakt gebracht wird, das das NCTA enthält, und wobei auf das biologische Produkt stromaufwärts und stromabwärts des Prozesses ein Verfahren zur Titration nach irgendeinem der Ansprüche 1 bis 14 angewendet wird, und die beiden Werte der erhaltenen Titer verglichen werden.
